# EUROPÄISCHE PATENTSCHRIFT

(11) **EP 1 414 357 B1**
(45) Veröffentlichungstag und Bekanntmachung des Hinweises auf die Patenterteilung: **01.12.2004**
(21) Anmeldenummer: 03735385.1
(22) Anmeldetag: 14.05.2003
(51) Int. Cl.: A61B 17/28

(54) **MEDIZINISCHES GREIF-UND HALTEINSTRUMENT**
MEDICAL GRASPING AND HOLDING INSTRUMENT
INSTRUMENT MEDICAL DE PRISE ET DE MAINTIEN

(30) Priorität: 01.06.2002 DE 10224500
(43) Veröffentlichungstag der Anmeldung: 06.05.2004
(73) Patentinhaber: Karl Storz GmbH & Co. KG, 78532 Tuttlingen (DE)
(72) Erfinder: FRANK, Tim, Wormit, Fife, KY 16 9TY (GB); GOVE, Jim, Dundee, DD4 6LS (GB)
(74) Vertreter: Hofmeister, Frank Horst
(86) Internationale Anmeldenummer: PCT/EP2003/005048
(87) Internationale Veröffentlichungsnummer: WO 2003/101315

(56) Entgegenhaltungen:
- US-A- 4 192 314
- US-A- 5 104 397
- US-A- 5 300 082
- US-A- 5 928 263
- US-A- 5 935 126

## Beschreibung

Die Erfindung betrifft ein medizinisches Greif- und Halteinstrument mit einer aus zwei Handgriffen bestehenden Handhabe und einem aus mindestens zwei Maulteilen bestehenden und über die Handhabe betätigbaren Halteteil, wobei die Maulteile des Halteteils über ein Federelement sowohl in einer das Halteteil freigebenden Endstellung als auch in einer das Halteteil verriegelnden Endstellung fixierbar sind.

Medizinische Greif- und Halteinstrumente werden für die verschiedensten Einsatzzwecke, beispielsweise als Zangen oder Nadelhalter verwendet. Diese bekannten Greif- und Halteinstrumente sind häufig zangenartig aufgebaut und weisen zwei um eine gemeinsame Schwenkachse verschwenkbare Hebelarme auf, deren distale Enden das Halteteil bilden und deren proximalen Enden die Handhabe bilden. Um das Halteteil in die Schließstellung zu überführen, ist es notwendig, die Handgriffe der Handhabe zusammenzudrücken und in dieser Stellung festzuhalten.

Die Notwendigkeit, die Handgriffe der Handhabe in der Schließstellung des Halteteils zusammengedrückt festhalten zu müssen, schränkt die Bewegungsfreiheit des Operateurs bei der Operation deutlich ein. Dieses Problem wird bei der HALS Operationstechnik (Hand Assisted Laparoscopic Surgery) noch verstärkt, bei der zusätzlich zum Einbringen des Laparoskops und gegebenenfalls laparoskopischer Instrumente in die Bauchhöhle ein Hautschnitt zum Einführen einer Hand des Operateurs geschaffen wird, damit der Operateur per Tastsinn und unter Beobachtung und Kontrolle durch das Laparoskop eine besser geführte Operation durchführen kann.

Während der Operation unterstützt der Operateur mit einer Hand in der Bauchhöhle des Patienten die Operation, in dem er beispielsweise ein Greif- und Halteinstrument wie einen Nadelhalter mit dieser in der Bauchhöhle befindlichen Hand führt und bedient. Die Möglichkeit die Operation zu unterstützen, wird aber deutlich eingeschränkt, wenn die Bewegungsfreiheit der Hand im wesentlichen darauf beschränkt ist, die Handgriffe der Handhabe zusammengedrückt zu halten, um ein vom Halteteil ergriffenes Teil, beispielsweise eine Nadel, nicht zu verlieren.

Aus der US 2 930 376 ist ein zangenähnliches Instrument mit zwei Handgriffen und einem aus zwei Maulteilen bestehenden Halteteil bekannt, wobei ein Maulteil einstückig starr mit einem Handgriff verbunden ist und das andere Maulteil um einen Gelenkpunkt verschwenkbar mit dem anderen Handgriff verbunden ist. Ferner weist dieses bekannte Instrument ein Federelement, das als Wendelfeder ausgebildet den starren Handgriff mit dem verschwenkbaren Maulteil verbindet, sowie einen Kniehebel auf, über den sich der verschwenkbare Handgriff an einer am starren Handgriff gelagerten Einstellschraube abstützt.

Der Kniehebel dient dabei als Widerlager für den verschwenkbaren Handgriff, damit beim Heranziehen des verschwenkbaren Handgriffs an den starren Handgriff das über die Wendelfeder in die Offenstellung vorgespannte Maulteil in eine geschlossene Stellung verlagerbar ist.

Dieses bekannte chirurgische Instrument weist einige konstruktive Nachteile auf, nämlich einerseits die Verwendung der Wendelfeder als Federelement, da Wendelfedern mit ihren unter Spannung aneinander liegenden Windungen sehr schlecht zu reinigen sind und andererseits der Einsatz des Kniehebels. Der Kniehebel stellt nicht nur ein zusätzlich zu fertigendes und zu montierendes Einzelteil dar, es besteht bei der bekannten Konstruktion zudem die große Gefahr, dass sich der Bediener des Instruments die Finger zwischen Kniehebel und Handgriff einklemmt, wenn der Handgriff in die geschlossene Stellung einschnappt.

Aus der US 5 104 397 ist ein anderes, gattungsgemässes Instrument bekannt, das über ein Federelement zwischen einer offenen, das Federelement entspannenden Endstellung, und einer geschlossenen, das Federelement spannenden Endstellung verlagerbar ist.

Davon ausgehend liegt der Erfindung die **Aufgabe** zugrunde, ein medizinisches Greif- und Halteinstrument der eingangs genannten Art zu schaffen, dass bei einfachem Aufbau sicher mit nur einer Hand zu bedienen ist und leicht reinigbar ist.

Die **Lösung** dieser Aufgabenstellung ist erfindungsgemäß dadurch gekennzeichnet, dass das als Blattfeder ausgebildete Federelement die beiden Handgriffe miteinander verbindend derart zwischen den Handgriffen der Handhabe angeordnet ist, dass das Federelement zum Fixieren der Maulteile in deren Endstellungen über einen Lagerpunkt eines Handgriffs am Federelement zwischen zwei das Federelement entspannenden Endstellungen verlagerbar ist.

Durch die erfindungsgemäße Ausbildung des medizinischen Instruments ist es möglich, das Instrument einhändig sicher und einfach zu führen und zu betätigen, da die Handgriffe bzw. die Maulteile des Halteteil mittels zwischen den Handgriffen angeordneten, als Blattfeder ausgebildeten Federelements in ihre Endstellungen überführbar sind, in denen die Handgriffe ohne das Aufbringen äußerer Kräfte gehalten werden. Ein solchermaßen ausgebildetes Instrument ist somit bestens auch für die HALS-Operationstechnik geeignet, bei der dem Operateur zum Bedienen eines medizinischen Instruments nur eine Hand zur Verfügung steht.

Der einfache Aufbau aus im einfachsten Fall nur drei Bauteilen, nämlich zwei mit jeweils einem Maulteil versehenen Handgriffen und einem die Handgriffe miteinander verbindenden Federelement, gewährleistet zudem eine kostengünstige Fertigung und gute Reinigbarkeit, zumal das Federelement als leicht und gründlich zu reinigende Blattfeder ausgebildet ist.

Gemäß einer praktischen Ausführungsform der Erfindung wird vorgeschlagen, dass das Federelement in Öffnungsrichtung der Handgriffe und somit der das Halteteil freigebenden Endstellung vorgespannt zwischen den Handgriffen angeordnet ist. Die Vorspannung des Federelements in Öffnungsrichtung der Handgriffe gewährleistet, dass ein versehentliches Betätigen des Halteteils und somit beispielsweise ein versehentliches Quetschen von Patientengewebe ausgeschlossen ist.

Weiterhin wird erfindungsgemäß vorgeschlagen, dass das Federelement mit einem Ende im Bereich des proximalen Endes eines Handgriffes und mit dem anderen Ende im mittleren Bereich des anderen Handgriffes gelagert ist. Durch dieses Auseinanderziehen der Lagerpunkte des Federelements an den Handgriffen wird die Druckkraft, die notwendig ist, um die Handgriffe zum Schließen des Halteteils zusammenzudrücken, reduziert.

Das Überführen der Handgriffe hin zur unteren, das Halteteil verriegelnden Endstellung kann erfindungsgemäß dadurch erleichtert werden, dass zur Lagerung des Federelements im mittleren Bereich des Handgriffes an diesem Handgriff ein in den Zwischenraum zwischen den beiden Handgriffen hineinragender Fortsatz ausgebildet ist, an dessen freiem Ende das Federelement gelagert ist.

Gemäß einer ersten praktischen Ausführungsform der Erfindung ist ein Handgriff der Handhabe einstückig starr mit einem Maulteil des Halteteils ausgebildet, während der andere Handgriff der Handhabe um einen Gelenkpunkt verschwenkbar mit dem anderen Maulteil des Halteteils verbunden ist. Bei dieser Ausgestaltungsform der Erfindung wird vorgeschlagen, dass das Federelement mit einem Ende im Bereich des proximalen Endes des starren Handgriffes und mit dem anderen Ende im mittleren Bereich des verschwenkbaren Handgriffes gelagert ist.

Aufgrund dieser Ausgestaltung ist es einfach und zuverlässig möglich, dass der Lagerpunkt, an dem das Federelement im mittleren Bereich des Handgriffes gelagert ist, in der das Halteteil verriegelnden unteren Endstellung unterhalb einer Linie angeordnet ist, die den Gelenkpunkt zwischen dem verschwenkbaren Handgriff und dem verschwenkbaren Maulteil mit dem Lagerpunkt des Federelements im Bereich des proximalen Endes des starren Handgriffes verbindet. Die Anordnung unterhalb dieser gedachten Linie bedeutet, dass eine äußere Kraft aufgewandt werden muß, um die Handgriffe wieder auseinander zu bewegen.

In der das Halteteil freigebenden oberen Endstellung ist dahingegen der Lagerpunkt, an dem das Federelement im mittleren Bereich des Handgriffes gelagert ist, oberhalb der Linie angeordnet ist, die den Gelenkpunkt zwischen dem verschwenkbaren Handgriff und dem verschwenkbaren Maulteil mit dem Lagerpunkt des Federelements im Bereich des proximalen Endes des starren Handgriffes verbindet.

Gemäß einer zweiten praktischen Ausfühungsform der Erfindung wird vorgeschlagen, dass beide Handgriffe der Handhabe einstückig starr mit jeweils einem Maulteil des Halteteils ausgebildet sind, wobei die Handgriffe bzw. Maulteile einander kreuzend um einen gemeinsamen Gelenkpunkt gegeneinander verschwenkbar gelagert sind. Bei diesem zangenartigen Aufbau bewirkt das Federelement je nach Lage des Lagerpunktes, an dem das Federelement im mittleren Bereich des einen Handgriffes gelagert ist zur Lage des Lagerpunktes, an dem das Federelement im proximalen Bereich des anderen Handgriffes gelagert ist, dass die Maulteile, abgesehen von einer labilen Zwischenlage, entweder hin zur geschlossenen Endstellung zusammengedrückt, oder aber hin zur offenen Endstellung auseinandergedrückt werden. Der Lagerpunkt, an dem das Federelement im mittleren Bereich des Handgriffes gelagert ist, ist bei dieser Ausführungsform auf einem Kreisbogen um den Gelenkpunkt angeordnet.

Mit einer dritten praktischen Ausführungsform der Erfindung wird vorgeschlagen, dass mindestens ein Maulteil des Halteteils fest mit jeweils einem Federelement verbunden ist, wobei das Federelement unter Vorspannung distalseitig zwischen dem Gelenkpunkt zum Verschwenken mindestens eines Maulteils und proximalseitig einem starren Widerlager so angeordnet ist, dass jedes Federelement über jeweils einen Handgriff der Handhabe zwischen in einer das Halteteil freigebenden Endstellung und in einer das Halteteil verriegelnden Endstellung verlagerbar ist.

Vorteilhafterweise ist bei dieser dritten erfindungsgemäßen Ausgestaltungsform ein Maulteil des Halteteils einstückig starr mit einem Handgriff der Handhabe ausgebildet und das andere Maulteil des Halteteils fest mit dem Federelement verbunden, wobei das Widerlager des Federelements starr mit dem starren Hangriff verbunden ist und der andere Handgriff der Handhabe um einen Gelenkpunkt verschwenkbar am proximalen Ende des starren Handgriffs gelagert ist und dieser verschwenkbare Handgriff mit dem Federelement verbunden ist.

Weiterhin wird mit der Erfindung vorgeschlagen, dass das Greifinstrument ein Nadelhalter, insbesondere ein Nadelhalter für die HALS-Operationstechnik, ist. Gerade für die HALS-Operationstechnik ist ein eine erfindungsgemäße Ausgestaltung vorteilhaft, da das Instrument einfach mit nur einer Hand zu bedienen ist.

Schließlich wird mit der Erfindung vorgeschlagen, dass das Greif- und Halteinstrument ein Rohrschaftinstrument ist, wobei die Handhabe über mindestens ein Kraftübertragungselement, insbesondere eine Zug-/Druckstange, mit dem Halteteil verbunden ist. Durch diese Ausgestaltung wird der Einsatzbereich der erfindungsgemäß ausgestalteten Handhabe zum Betätigen eines Halteteils deutlich erweitert, da das in den beiden Endstellung fixierbare Halteteil nicht in der Art einer Zange unmittelbar mit den Handgriffen der Handhabe verbunden sein muß.

Weitere Merkmale und Vorteile der Erfindung ergeben sich anhand der nachfolgenden Beschreibung der zugehörigen Zeichnung, in der vier Ausführungsbeispiele eines erfindungsgemäßen medizinischen Greif- und Halteinstruments nur beispielhaft dargestellt sind. In der Zeichnung zeigt:
- Fig. 1a: eine Seitenansicht einer ersten Ausführungsform eines erfindungsgemäßen medizinischen Instruments, das Halteteil in der offenen Endstellung darstellend;
- Fig. 1b eine Fig. 1a: entsprechende Darstellung, jedoch das medizinische Instrument in einer Haltestellung darstellend;
- Fig. 1c: eine Fig. 1a und 1b entsprechende Darstellung, jedoch das Halteteil in der geschlossenen Endstellung darstellend;
- Fig. 2a: eine Seitenansicht einer zweiten Ausführungsform eines erfindungsgemäßen medizinischen Instruments, das Halteteil in der offenen Endstellung darstellend;
- Fig. 2b: eine Fig. 2a entsprechende Darstellung, jedoch das Halteteil in der geschlossenen Endstellung darstellend;
- Fig. 3a: eine Seitenansicht einer dritten Ausführungsform eines erfindungsgemäßen medizinischen Instruments, das Halteteil in der offenen Endstellung darstellend;
- Fig. 3b: eine Fig. 3a entsprechende Darstellung, jedoch das Halteteil in der geschlossenen Endstellung darstellend und
- Fig. 4: eine schematische Seitenansicht einer vierten Ausführungsform eines erfindungsgemäßen medizinischen Instruments.

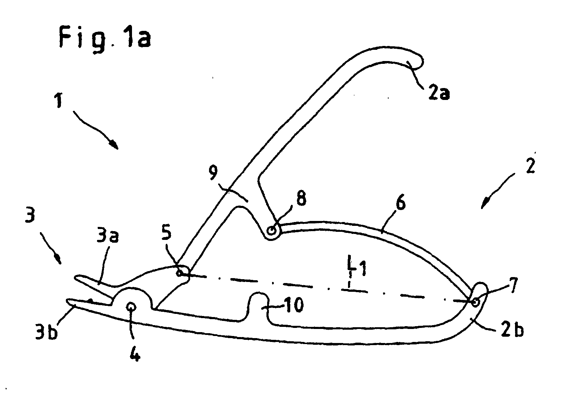

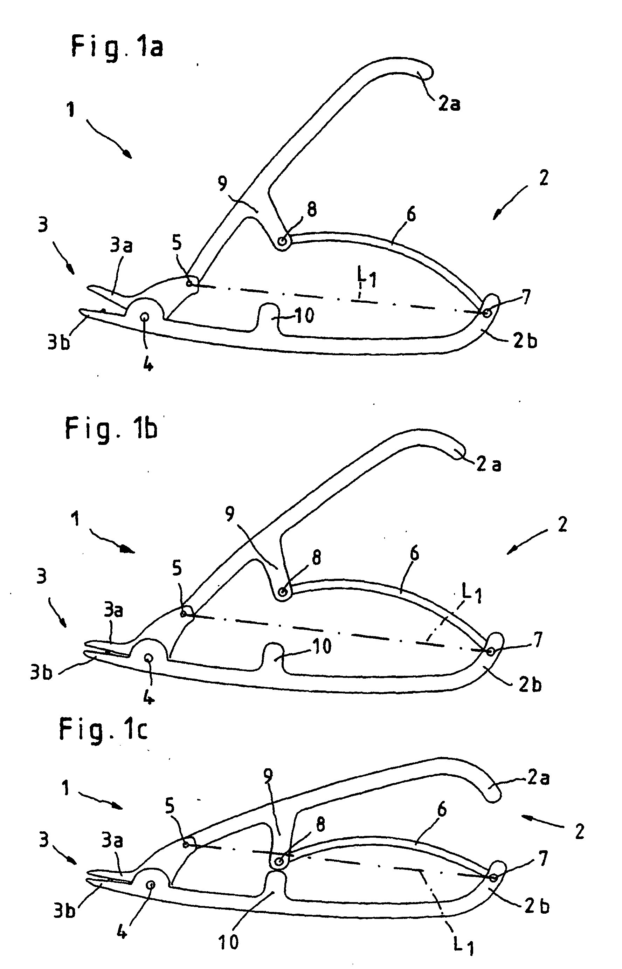

Bei den in den Abbildungen Fig. 1a bis 3c dargestellten medizinischen Greif- und Halteinstrumenten handelt es sich um chirurgische Nadelhalter 1, insbesondere zur Verwendung in der laparoskopischen Chirurgie nach der HALS-Operationstechnik.

Die prinzipiell zangenartig aufgebauten Nadelhalter 1 bestehen im wesentlichen aus einer proximalseitigen Handhabe 2 mit zwei Handgriffen 2a und 2b sowie einem distalseitigen Halteteil 3 mit zwei Maulteilen 3a und 3b, die über einen Gelenkpunkt 4 relativ zueinander verschwenkbar sind.

Bei der in den Abbildungen Fig. 1a bis 1c dargestellten ersten Ausführungsform des Nadelhalters 1 sind der Handgriff 2b sowie das Maulteil 3b einstückig starr miteinander verbunden, wohingegen der andere Handgriff 2a sowie das andere Maulteil 3a über einen Gelenkpunkt 5 verschwenkbar miteinander verbunden sind. Bei dieser dargestellten Ausführungsform kreuzen sich die Maulteile 3a, 3b bzw. die Handgriffe 2a, 2b nicht.

Wie aus den Abbildungen Fig. 1a bis 1c weiterhin ersichtlich, ist zwischen den Handgriffen 2a und 2b der Handhabe 2 ein als Blattfeder ausgebildetes Federelement 6 angeordnet, welches mit einem Ende an einem Lagerpunkt 7 im Bereich des proximalen Endes des starren Handgriffes 2b und mit dem anderen Ende an einem Lagerpunkt 8 im mittleren Bereich des verschwenkbaren Handgriffes 2a gelagert ist. Die Lagerung des Federelements 6 im mittleren Bereich des verschwenkbaren Handgriffes 2a erfolgt bei der dargestellten Ausführungsform des Nadelhalters 1 am freien Ende eines am verschwenkbaren Handgriff 2a angeordneten, in den Zwischenraum zwischen den beiden Handgriffen 2a, 2b hineinragenden Fortsatz 9.

Die Bedienung dieses in den Abbildungen Fig. 1a bis 1c dargestellten medizinischen Greif- und Halteinstruments geschieht wie folgt:

Bei der HALS-Operationstechnik führt der Operateur den in der Handinnenfläche geschützt gehaltenen Nadelhalter 1 zusammen mit seiner Hand durch einen geeigneten Hautschnitt in die Bauchhöhle des Patienten ein, wobei der Nadelhalter 1 sich möglichst in der in Fig. 1c dargestellten Stellung befindet, da diese die geringste Baugröße aufweist.

In der in Fig. 1a dargestellten offenen Endstellung des Halteteils 3 werden die Handgriffe 2a und 2b durch das in Öffnungsrichtung der Handgriffe 2a, 2b vorgespannte Federelement 6 auseinander gedrückt. Diese Stellung, in der der Lagerpunkt 8, an dem das Federelement 6 im mittleren Bereich des Handgriffes 2a gelagert ist, oberhalb einer Linie L₁ angeordnet ist, die den Gelenkpunkt 5 zwischen dem verschwenkbaren Handgriff 2a und dem verschwenkbaren Maulteil 3a mit dem Lagerpunkt 7 des Federelements 6 im Bereich des proximalen Endes des starren Handgriffes 2b verbindet, stellt eine Endstellung der Handgriffe 2a, 2b der Handhabe 2 bzw. der Maulteile 3a, 3b des Halteteils 3 dar, in der das Federelement 6 die Handgriffe 2a, 2b bzw. die Maulteile 3a, 3b fixiert, da die Handgriffe 2a, 2b aus dieser Endstellung nur durch das Aufbringen einer äußeren, gegen die Federkraft des Federelements 6 wirkende Kraft verstellbar sind.

Um nun eine Nadel zu ergreifen, drückt der Operateur die Handgriffe 2a, 2b gegen die Kraft des Federelements 6 aufeinander zu zusammen, bis der Nadelhalter 1 die in Fig. 1b dargestellte Stellung einnimmt, in der die Maulteile 3a, 3b des Halteteils 3 die Nadel haltend geschlossen sind. In dieser Stellung befindet sich der Lagerpunkt 8 auf oder etwas oberhalb der Linie L₁, die den Gelenkpunkt 5 mit dem Lagerpunkt 7 verbindet.

Ein weiteres Zusammendrücken der Handgriffe 2a, 2b bewirkt, dass das Federelement 6 weiter durchgebogen wird, bis der Lagerpunkt 8 die Linie L₁, die den Gelenkpunkt 5 mit dem Lagerpunkt 7 verbindet, überspringt, da die Verbindung Handgriff 2a/Maulteil 3a im Gelenkpunkt 5 einknickt und die in Fig. 1 c dargestellte zweite Endstellung einnimmt, in der die Handgriffe 2a, 2b zusammengedrückt und die Maulteile 3a, 3b des Halteteils 3 geschlossen sind. Da sich das Federelement 6 beim Überspringen der Linie L₁ wieder ein wenig entspannt, sind die Handgriffe 2a, 2b in dieser Endstellung wiederum fixiert, da es dem Aufbringen einer äußeren Kraft bedarf, um die Handgriffe 2a, 2b gegen die Federkraft des Federelements 6 wieder auseinanderzudrücken.

Die Bewegung der Handgriffe 2a, 2b aufeinander zu wird durch einen am starren Handgriff 2b ausgebildeten Anschlag 10 begrenzt, gegen den der am verschwenkbaren Handgriff 2a angeordnete Fortsatz 9 anläuft.

Zum Öffnen der Handgriffe 2a, 2b bzw. der Maulteile 3a, 3b führt der Operateur einen oder mehrere Finger seiner Hand zwischen die Handgriffe 2a, 2b und drückt die Handgriffe 2a, 2b gegen die Federkraft des Federelements 6 wieder auseinander.

Aufgrund der Fixierung des Nadelhalters 1 in der geschlossenen Stellung des Halteteils 3 kann der Operateur den Griff um die Handgriffe 2a, 2b der Handhabe 2 lösen, ohne das die Gefahr besteht, dass die von den Maulteilen 3a, 3b ergriffene Nadel verrutscht oder gar aus dem Halteteil 3 herausfällt.

Ein solchermaßen ausgebildetes medizinisches Greif- und Halteinstrument ist somit insbesondere für die HALS-Operationstechnik bestens geeignet, da es mit nur einer Hand einfach und sicher zu bedienen ist und durch das Federelement 5 in seinen Endstellungen arretierend gehalten wird.

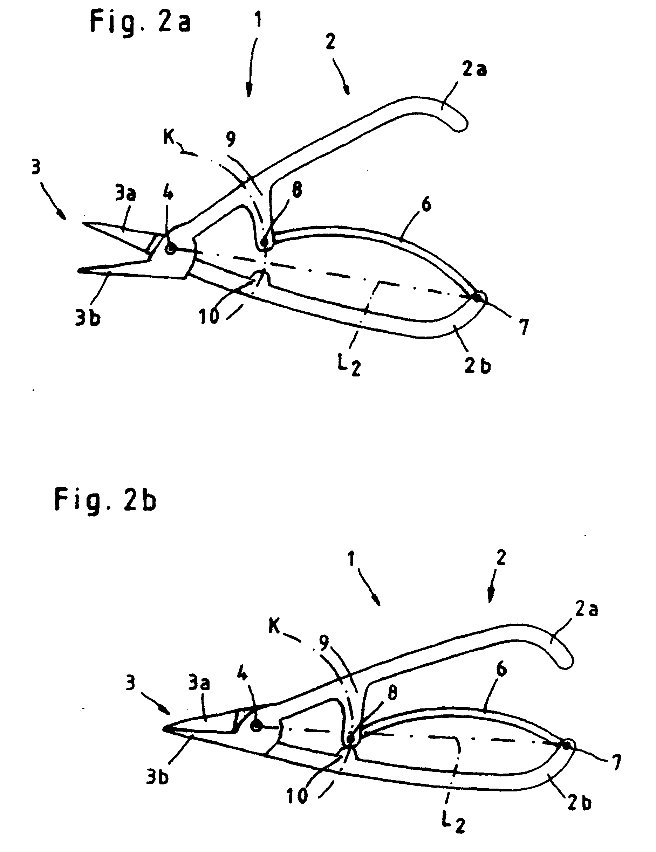

Die in den Abbildungen Fig. 2a und 2b dargestellte zweite Ausführungsform des Nadelhalter 1 unterscheidet sich von der zuvor anhand der Abbildungen Fig. 1a bis 1c beschriebenen ersten Ausführungsform dadurch, dass beide Handgriffe 2a, 2b der Handhabe 2 starr mit den jeweiligen Maulteilen 3a, 3b des Halteteils 3 verbunden sind. Bei diesem zangenartigen Aufbau des Nadelhalters 1 kreuzen sich die Maulteile 3a, 3b bzw. Handgriffe 2a, 2b im Gelenkpunkt 4, um den die Maulteile 3a, 3b bzw. Handgriffe 2a, 2b relativ zueinander verschwenkbar sind.

Auch bei dieser zweiten Ausführungsform des Nadelhalters 1 ist, wie aus Fig. 2a und 2b ersichtlich, zwischen den Handgriffen 2a und 2b der Handhabe 2 ein als Blattfeder ausgebildetes Federelement 6 angeordnet, welches mit einem Ende an einem Lagerpunkt 7 im Bereich des proximalen Endes des einen Handgriffes 2b und mit dem anderen Ende an einem Lagerpunkt 8 im mittleren Bereich des anderen Handgriffes 2a gelagert ist. Die Lagerung des Federelements 6 im mittleren Bereich des verschwenkbaren Handgriffes 2a erfolgt auch bei dieser dargestellten zweiten Ausführungsform des Nadelhalters 1 am freien Ende eines am Handgriff 2a angeordneten, in den Zwischenraum zwischen den beiden Handgriffen 2a, 2b hineinragenden Fortsatz 9.

Die Bedienung dieses Nadelhalters 1 geschieht prinzipiell in der gleichen Art und Weise, wie diese zuvor zur ersten Ausführungsform gemäß den Abbildungen Fig. 1a bis 1c beschrieben wurde.

Das Fixieren des Federelements 6 in seinen beiden Endstellungen erfolgt bei der zweiten Ausführungsform jedoch dadurch, dass der Lagerpunkt 8, an dem das Federelement 6 im mittleren Bereich des Handgriffes 2a gelagert ist, auf einem Kreisbogen K um den Gelenkpunkt 4 angeordnet ist.

Beim Zusammendrücken der Handgriffe 2a, 2b, ausgehend von der offenen Endstellung gemäß Fig. 2a, hin zur geschlossenen Endstellung gemäß Fig. 2b wird das Federelement 6 entgegen seiner Federkraft weiter durchgebogen, da sich der Abstand zwischen den Lagerpunkten 8 und 7 zunächst verringert, bis die Kreisbahn K eine Linie L₂ schneidet, die den Gelenkpunkt 4 mit dem Lagerpunkt 7 des Federelements 6 im Bereich des proximalen Endes des Handgriffes 2b schneidet Sobald die Handgriffe 2a, 2b weiter zusammengedrückt werden, entspannt sich das Federelement 6 wieder, da der Abstand zwischen den Lagerpunkten 7 und 8 wieder größer wird. Da sich das Federelement 6 beim Überqueren der Linie L₂ wieder entspannt, sind die Handgriffe 2a, 2b in dieser Endstellung wiederum fixiert, da es dem Aufbringen einer äußeren Kraft bedarf, um die Handgriffe 2a, 2b gegen die Federkraft des Federelements 6 wieder auseinanderzudrücken.

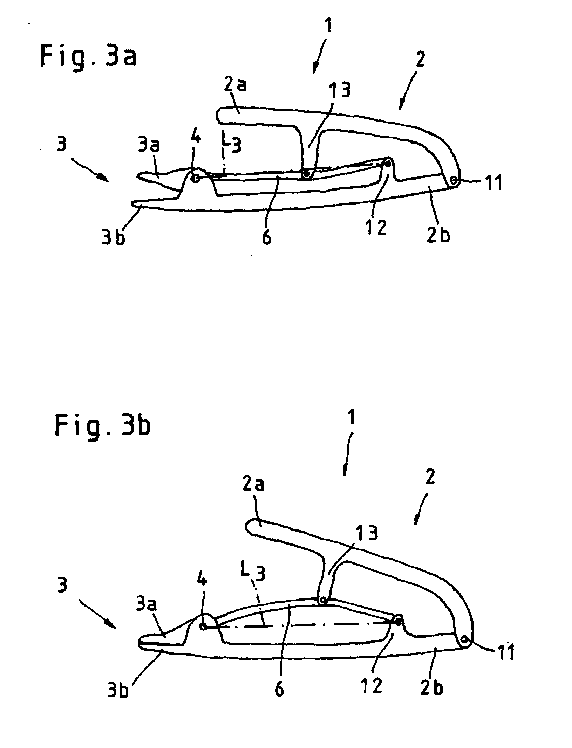

Die Abbildungen Fig. 3a und 3c zeigen eine dritte Ausführungsform des Nadelhalters 1. Bei dieser Ausführungsform weist der Nadelhalter 1 gegenüber den in den Abbildungen Fig. 1a bis 2b dargestellten Ausführungsformen einen deutlich unterschiedlichen Aufbau auf. Während bei den ersten und zweiten Ausführungsformen die Maulteile 3a und 3b jeweils direkt mit den Handgriffen 2a und 2b verbunden sind, ist bei der in Fig. 3a und 3b dargestellten dritten Ausführungsform der Handgriff 2a nur indirekt, nämlich über das Federelement 6, mit dem Maulteil 3a verbunden.

Wie aus Fig. 3a und 3b ersichtlich, sind der Handgriff 2b sowie das Maulteil 3b einstückig starr miteinander verbunden. Der andere Handgriff 2a ist um einen Gelenkpunkt 11 verschwenkbar am proximalen Ende des starren Handgriffs 3b gelagert. Die Verbindung zwischen dem zweiten Maulteil 3a und dem Handgriff 2a erfolgt über das Federelement 6, das fest mit dem Maulteil 3a verbunden ist. Das Federelement 6 ist unter Vorspannung zwischen distalseitig dem Gelenkpunkt 4 zum Verschwenken mindestens eines der beiden Maulteile 3a, 3b und proximalseitig einem am starren Handgriff 2b angeordneten starren Widerlager 12 so angeordnet dass das Federelement 6 über den Handgriff 2a der Handhabe 2 zwischen in einer das Halteteil 3 freigebenden Endstellung gemäß Fig. 3a und in einer das Halteteil 3 verriegelnden Endstellung gemäß Fig. 3b verlagerbar ist. Das Verlagern des Federelements 6 über den Handgriff 2a erfolgt über einen Verbindungssteg 13 zwischen dem Handgriff 2a und dem Federelement 6.

In der in Fig. 3a dargestellten offenen Endstellung wird das Federelement 6 durch den Handgriff 2a nach unten durchgedrückt, was ein Öffnen des Halteteils 3 bewirkt. Das Durchdrücken des Federelements 6 unter eine Linie L₃, die die beiden Einspannpunkte des Federelements 6 am Gelenkpunkt 4 und am Widerlager 12 miteinander verbindet bewirkt, dass die Handgriffe 2a, 2b in dieser Endstellung fixiert sind, da es dem Aufbringen einer äußeren Kraft bedarf, um die Handgriffe 2a, 2b gegen die Federkraft des Federelements 6 wieder auseinanderzuziehen.

Zum Überführen des Halteteils 3 in die in Fig. 3b dargestellte geschlossene Endstellung muß der Handgriff 2a entgegen der Federkraft des Federelements 6 angehoben werden, bis das Federelement 6 sich die Linie L₃ überspringend nach oben durchbiegt.

Alternativ zu dieser dargestellten dritten Ausführungsform ist es möglich, diese Anordnung des Federelements 6 auch so auszugestalten, dass beide Maulteile 3a, 3b fest mit jeweils einem Federelement 6 verbunden sind und nur indirekt über die auf die Federelemente 6 einwirkenden Handgriffe 2a, 2b betätigbar sind. Bei dieser nicht dargestellten Ausführungsform bedarf es eines starren Mittelstegs, an dessen distalem Ende die Maulteile 3a, 3b um den Gelenkpunkt 4 verschwenkbar sind und an dessen proximalem Ende die Handgriffe 2a, 2b um den Gelenkpunkt 11 verschwenkbar gelagert sind. Außerdem dient der starre Mittelsteg zur Aufnahme der beiden Widerlager 12 für die zwei Federelemente 6.

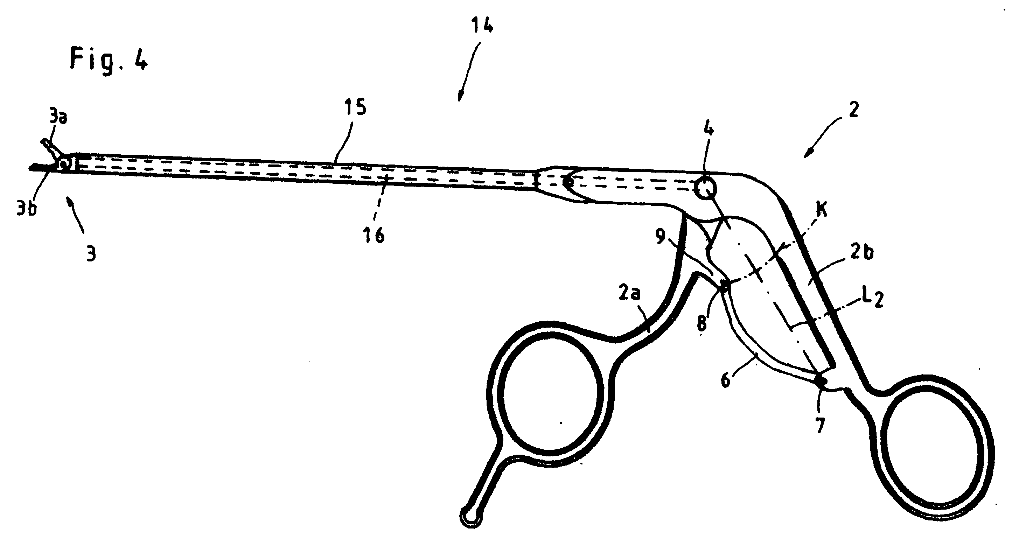

Fig. 4 zeigt schließlich eine vierte Ausführungsform zur Ausgestaltung eines medizinischen Greif- und Halteinstruments. Bei diesem dargestellten medizinischen Instrument handelt es sich um ein Rohrschaftinstrument 14 mit einem vorzugsweise hohlzylindrischen Schaft 15, an dessen proximalem Ende die aus den Handgriffen 2a und 2b bestehende Handhabe 2 und an dessen distalem Ende das aus den beiden Maulteilen 3a und 3b bestehende Halteteil 3 angeordnet ist. Zum Betätigen des Halteteils 3 über die Handhabe 2 sind die Handhabe 2 und das Halteteil 3 über mindestens ein Kraftübertragungselement miteinander verbunden. Bei der dargestellten Ausführungsform ist das Kraftübertragungselement als Zug-/Druckstange 16 ausgebildet.

Alternativ ist es auch möglich, das Kraftübertragungselement beispielsweise als Bowdenzug auszugestalten.

Die Handhaben 2 zum Betätigen des Halteteils 3 können bei der Ausbildung des Greif- und Halteinstruments als Rohrschaftinstrument 14 so ausgebildet sein, wie dies voranstehend zu den Abbildungen Fig. 1a bis 3b beschrieben wurde. In allen Fällen ermöglicht die Anordnung des wenigstens einen Federelements 6, dass das Halteteil 3 sowohl in einer offenen Endstellung als auch in einer geschlossenen Endstellung fixierbar ist.

### Bezugszeichenliste

- 1: Nadelhalter
- 2: Handhabe
- 2a: (verschwenkbarer) Handgriff
- 2b: (starrer) Handgriff
- 3: Halteteil
- 3a: (verschwenkbares) Maulteil
- 3b: (starres) Maulteil
- 4: Gelenkpunkt
- 5: Gelenkpunkt
- 6: Federelement
- 7: Lagerpunkt
- 8: Lagerpunkt
- 9: Fortsatz
- 10: Anschlag
- 11: Gelenkpunkt
- 12: Widerlager
- 13: Verbindungssteg
- 14: Rohrschaftinstrument
- 15: Schaft
- 16: Zug-/Druckstange

- L₁: Linie
- L₂: Linie
- L₃: Linie
- K: Kreisbogen

## Patentansprüche

1. Medizinisches Greif- und Halteinstrument mit einer aus zwei Handgriffen (2a, 2b) bestehenden Handhabe (2) und einem aus mindestens zwei Maulteilen (3a, 3b) bestehenden und über die Handhabe (2) betätigbaren Halteteil (3), wobei die Maulteile (3a, 3b) des Halteteils (3) über ein Federelement (6) sowohl in einer das Halteteil (3) freigebenden Endstellung als auch in einer das Halteteil (3) verriegelnden Endstellung fixierbar sind,
**dadurch gekennzeichnet,**
**dass** das als Blattfeder ausgebildete Federelement (6) die beiden Handgriffe (2a, 2b) miteinander verbindend derart zwischen den Handgriffen (2a, 2b) der Handhabe (2) angeordnet ist, dass das Federelement (6) zum Fixieren der Maulteile (3a, 3b) in deren Endstellungen über einen Lagerpunkt (8) eines Handgriffs (2a oder 2b) am Federelement (6) zwischen zwei das Federelement (6) entspannenden Endstellungen verlagerbar ist.

2. Medizinisches Greif- und Halteinstrument nach Anspruch 1, **dadurch gekennzeichnet, dass** das Federelement (6) in Öffnungsrichtung der Handgriffe (2a, 2b) und somit der das Halteteil (3) freigebenden Endstellung vorgespannt zwischen den Handgriffen (2a, 2b) angeordnet ist.

3. Medizinisches Greif- und Halteinstrument nach Anspruch 1 oder 2, **dadurch gekennzeichnet, dass** das Federelement (6) mit einem Ende an einem Lagerpunkt (7) im Bereich des proximalen Endes eines Handgriffes (2b) und mit dem anderen Ende an einem Lagerpunkt (8) im mittleren Bereich des anderen Handgriffes (2a) gelagert ist.

4. Medizinisches Greif- und Halteinstrument nach Anspruch 3, **dadurch gekennzeichnet, dass** zur Lagerung des Federelements (6) im mittleren Bereich des anderen Handgriffes (2a) an diesem Handgriff (2a) ein in den Zwischenraum zwischen den beiden Handgriffen (2a, 2b) hineinragender Fortsatz (9) ausgebildet ist, an dessen freiem Ende das Federelement (6) gelagert ist.

5. Medizinisches Greif- und Halteinstrument nach einem der Ansprüche 1 bis 4, **dadurch gekennzeichnet, dass** ein Handgriff (2b) der Handhabe (2) einstückig starr mit einem Maulteil (3b) des Halteteils (3) ausgebildet ist, während der andere Handgriff (2a) der Handhabe (2) um einen Gelenkpunkt (5) verschwenkbar mit dem anderen Maulteil (3a) des Halteteils (3) verbunden ist.

6. Medizinisches Greif- und Halteinstrument nach Anspruch 5, **dadurch gekennzeichnet, dass** das Federelement (6) mit einem Ende am Lagerpunkt (7) im Bereich des proximalen Endes des starren Handgriffes (2b) und mit dem anderen Ende am Lagerpunkt (8) im mittleren Bereich des verschwenkbaren Handgriffes (2a) gelagert ist.

7. Medizinisches Greif- und Halteinstrument nach Anspruch 5 oder 6, **dadurch gekennzeichnet, dass** der Lagerpunkt (8), an dem das Federelement (6) im mittleren Bereich des verschwenkbaren Handgriffes (2a) gelagert ist, in der das Halteteil (3) freigebenden oberen Endstellung oberhalb einer Linie (L₁) angeordnet ist, die den Gelenkpunkt (5) zwischen dem verschwenkbaren Handgriff (2a) und dem verschwenkbaren Maulteil (3a) mit dem Lagerpunkt (7) des Federelements (6) im Bereich des proximalen Endes des starren Handgriffes (2b) verbindet.

8. Medizinisches Greif- und Halteinstrument nach einem der Ansprüche 5 bis 7, **dadurch gekennzeichnet, dass** der Lagerpunkt (8), an dem das Federelement (6) im mittleren Bereich des verschwenkbaren Handgriffes (2a) gelagert ist, in der das Halteteil (3) verriegelnden unteren Endstellung unterhalb einer Linie (L₁) angeordnet ist, die den Gelenkpunkt (5) zwischen dem verschwenkbaren Handgriff (2a) und dem verschwenkbaren Maulteil (3a) mit dem Lagerpunkt (7) des Federelements (6) im Bereich des proximalen Endes des starren Handgriffes (2b) verbindet.

9. Medizinisches Greif- und Halteinstrument nach einem der Ansprüche 1 bis 4, **dadurch gekennzeichnet, dass** beide Handgriffe (2a, 2b) der Handhabe (2) einstückig starr mit jeweils einem Maulteil (3a, 3b) des Halteteils (3) ausgebildet sind, wobei die Handgriffe (2a, 2b) bzw. Maulteile (3a, 3b) einander kreuzend um einen gemeinsamen Gelenkpunkt (4) gegeneinander verschwenkbar gelagert sind.

10. Medizinisches Greif- und Halteinstrument nach Anspruch 9, **dadurch gekennzeichnet, dass** der Lagerpunkt (8), an dem das Federelement (6) im mittleren Bereich eines Handgriffes (2a) gelagert ist, auf einem Kreisbogen (K) um den Gelenkpunkt (4) angeordnet ist.

11. Medizinisches Greif- und Halteinstrument nach Anspruch 1, **dadurch gekennzeichnet, dass** mindestens ein Maulteil (3a, 3b) des Halteteils (3) fest mit jeweils einem Federelement (6) verbunden ist, wobei jedes Federelement (6) unter Vorspannung zwischen distalseitig dem Gelenkpunkt (4) zum Verschwenken mindestens eines Maulteils (3a, 3b) und proximalseitig einem starren Widerlager (12) so angeordnet ist, dass jedes Federelement (6) über jeweils einen Handgriff (2a, 2b) der Handhabe (2) zwischen in einer das Halteteil (3) freigebenden Endstellung und in einer das Halteteil (3) verriegelnden Endstellung venagerbar ist.

12. Medizinisches Greif- und Halteinstrument nach Anspruch 11, **dadurch gekennzeichnet, dass** ein Maulteil (3b) des Halteteils (3) einstückig starr mit einem Handgriff (2b) der Handhabe (2) ausgebildet ist und das andere Maulteil (3a) des Halteteils (3) fest mit dem Federelement (6) verbunden ist, wobei das Widerlager (12) des Federelements (6) starr mit dem starren Handgriff (2b) verbunden ist und der andere Handgriff (2a) der Handhabe (2) um einen Gelenkpunkt (11) verschwenkbar am proximalen Ende des starren Handgriffs (2b) gelagert ist und der verschwenkbare Handgriff (2a) mit dem Federelement (6) verbunden ist.

13. Medizinisches Greif- und Halteinstrument nach einem der Ansprüche 1 bis 12 **dadurch gekennzeichnet, dass** das Greif- und Halteinstrument ein Nadelhalter (1) ist.

14. Medizinisches Greif- und Halteinstrument nach Anspruch 1, **dadurch gekennzeichnet, dass** das Greif- und Halteinstrument ein Rohrschaftinstrument (14) ist, wobei die Handhabe (2) über mindestens ein Kraftübertragungselement, insbesondere eine Zug-/Druckstange (16), mit dem Halteteil (3) verbunden ist.

## Claims

1. Medical gripping and holding instrument, with a handle (2) consisting of two handgrips (2a, 2b) and with a holding part (3) which consists of at least two jaw parts (3a, 3b) and which can be operated via the handle (2), the jaw parts (3a, 3b) of the holding part (3) being able to be fixed via a spring element (6) both in an end position freeing the holding part (3) and also in an end position locking the holding part (3), **characterized in that** the spring element (6) designed as a leaf spring and connecting the two handgrips (2a, 2b) to one another is arranged between the handgrips (2a, 2b) of the handle (2) in such a way that the spring element (6) for fixing the jaw parts (3a, 3b) in their end positions can be displaced via a bearing point (8) of one handgrip (2a or 2b) on the spring element (6) between two end positions releasing the spring element (6).

2. Medical gripping and holding instrument according to Claim 1, **characterized in that** the spring element (6) arranged between the handgrips (2a, 2b) is pretensioned in the opening direction of the handgrips (2a, 2b) and thus in the direction of the end position freeing the holding part (3).

3. Medical gripping and holding instrument according to Claim 1 or 2, **characterized in that** the spring element (6) is mounted with one end at a bearing point (7) in the area of the proximal end of one handgrip (2b), and with the other end at a bearing point (8) in the middle area of the other handgrip (2a).

4. Medical gripping and holding instrument according to Claim 3, **characterized in that**, in order to mount the spring element (6) in the middle area of the other handgrip (2a), a projection (9) is formed on this handgrip (2a), which projection (9) protrudes into the space between the two handgrips (2a, 2b), and the spring element (6) is mounted at the free end of said projection (9).

5. Medical gripping and holding instrument according to one of Claims 1 to 4, **characterized in that** one handgrip (2b) of the handle (2) is designed rigidly in one piece with one jaw part (3b) of the holding part (3), while the other handgrip (2a) of the handle (2) is connected to the other jaw part (3a) of the holding part (3) so as to be pivotable about a hinge point (5).

6. Medical gripping and holding instrument according to Claim 5, **characterized in that** the spring element (6) is mounted with one end at the bearing point (7) in the area of the proximal end of the rigid handgrip (2b), and with the other end at the bearing point (8) in the middle area of the pivotable handgrip (2a).

7. Medical gripping and holding instrument according to Claim 5 or 6, **characterized in that** the bearing point (8), at which the spring element (6) is mounted in the middle area of the pivotable handgrip (2a), is arranged, in the upper end position freeing the holding part (3), above a line (L₁) which connects the hinge point (5) between the pivotable handgrip (2a) and the pivotable jaw part (3a) to the bearing point (7) of the spring element (6) in the area of the proximal end of the rigid handgrip (2b).

8. Medical gripping and holding instrument according to one of Claims 5 to 7, **characterized in that** the bearing point (8), at which the spring element (6) is mounted in the middle area of the pivotable handgrip (2a), is arranged, in the lower end position locking the holding part (3), below a line (L₁) which connects the hinge point (5) between the pivotable handgrip (2a) and the pivotable jaw part (3a) to the bearing point (7) of the spring element (6) in the area of the proximal end of the rigid handgrip (2b).

9. Medical gripping and holding instrument according to one of Claims 1 to 4, **characterized in that** both handgrips (2a, 2b) of the handle (2) are designed rigidly and in one piece with in each case one jaw part (3a, 3b) of the holding part (3), the handgrips (2a, 2b) and jaw parts (3a, 3b) intersecting one another and mounted pivotably with respect to one another about a common hinge point (4).

10. Medical gripping and holding instrument according to Claim 9, **characterized in that** the bearing point (8), at which the spring element (6) is mounted in the middle area of one handgrip (2a), is arranged on an arc of a circle (K) around the hinge point (4).

11. Medical gripping and holding instrument according to Claim 1, **characterized in that** at least one jaw part (3a, 3b) of the holding part (3) is fixedly connected to in each case one spring element (6), each spring element (6) being arranged, with pretensioning, between at the distal end the hinge point (4) for pivoting at least one jaw part (3a, 3b) and at the proximal end a rigid abutment (12), in such a way that each spring element (6) can be displaced via in each case one handgrip (2a, 2b) of the handle (2) between an end position freeing the holding part (3) and an end position locking the holding part (3).

12. Medical gripping and holding instrument according to Claim 11, **characterized in that** one jaw part (3b) of the holding part (3) is designed rigidly and in one piece with one handgrip (2b) of the handle (2), and the other jaw part (3a) of the holding part (3) is connected fixedly to the spring element (6), the abutment (12) of the spring element (6) being connected rigidly to the rigid handgrip (2b), and the other handgrip (2a) of the handle (2) is mounted pivotably about a hinge point (11) at the proximal end of the rigid handgrip (2b), and the pivotable handgrip (2a) is connected to the spring element (6).

13. Medical gripping and holding instrument according to one of Claims 1 to 12, **characterized in that** the gripping and holding instrument is a needle holder (1).

14. Medical gripping and holding instrument according to Claim 1, **characterized in that** the gripping and holding instrument is a tubular shaft instrument (14), the handle (2) being connected to the holding part (3) via at least one force transmission element, in particular a pull/push rod (16).

## Revendications

1. Instrument médical de prise et de maintien, comportant une manette (2) constituée par deux poignées (2a, 2b) et une partie de maintien (3) constituée par au moins deux mâchoires (3a, 3b) et actionnable via la manette (2), les mâchoires (3a, 3b) de la partie de maintien (3) pouvant être fixées via un élément ressort (6) tant dans une position terminale libérant la partie de maintien (3) que dans une position terminale verrouillant la partie de maintien (3),
**caractérisé en ce que**
l'élément ressort (6), réalisé sous forme de ressort à lame reliant les deux poignées (2a, 2b) l'une à l'autre, est agencé entre les poignées (2a, 2b) de la manette (2) de telle sorte que pour fixer les mâchoires (3a, 3b) dans leurs positions terminales via un point de montage (8) d'une poignée (2a ou 2b) sur l'élément ressort (6), l'élément ressort (6) peut être déplacé entre deux positions terminales relâchant l'élément ressort (6).

2. Instrument médical de prise et de maintien selon la revendication 1, **caractérisé en ce que** l'élément ressort (6) est agencé précontraint entre les poignées (2a, 2b) dans le sens d'ouverture des poignées (2a, 2b) et par conséquent de la position terminale libérant la partie de maintien (3).

3. Instrument médical de prise et de maintien selon l'une ou l'autre des revendications 1 et 2, **caractérisé en ce que** l'élément ressort (6) est monté par une extrémité sur un point de montage (7) dans la région de l'extrémité proximale d'une poignée (2b) et par l'autre extrémité sur un point de montage (8) dans la région médiane de l'autre poignée (2a).

4. Instrument médical de prise et de maintien selon la revendication 3, **caractérisé en ce que** pour monter l'élément ressort (6) dans la région médiane de l'autre poignée (2a), un prolongement (9) faisant saillie dans l'espace intermédiaire entre les deux poignées (2a, 2b) est réalisé sur cette poignée (2a), prolongement à l'extrémité libre duquel est monté l'élément ressort (6).

5. Instrument médical de prise et de maintien selon l'une des revendications 1 à 4, **caractérisé en ce qu'**une poignée (2b) de la manette (2) est réalisée d'un seul tenant et de manière rigide avec une mâchoire (3b) de la partie de maintien (3), tandis que l'autre poignée (2a) de la manette (2) est reliée à l'autre mâchoire (3a) de la partie de maintien (3) de manière à pouvoir pivoter autour d'un point d'articulation (5).

6. Instrument médical de prise et de maintien selon la revendication 5, **caractérisé en ce que** l'élément ressort (6) est monté par une extrémité sur le point de montage (7) dans la région de l'extrémité proximale de la poignée (2b) rigide, et par l'autre extrémité sur le point de montage (8) dans la région médiane de la poignée (2a) pivotable.

7. Instrument médical de prise et de maintien selon l'une ou l'autre des revendications 5 et 6, **caractérisé en ce que** le point de montage (8), sur lequel est monté l'élément ressort (6) dans la région médiane de la poignée (2a) pivotable, est agencé, dans la position terminale supérieure libérant la partie de maintien (3), au-dessus d'une ligne (L₁) qui relie le point d'articulation (5) entre la poignée (2a) pivotable et la mâchoire (3a) pivotable avec le point de montage (7) de l'élément ressort (6) dans la région de l'extrémité proximale de la poignée (2b) rigide.

8. Instrument médical de prise et de maintien selon l'une des revendications 5 à 7, **caractérisé en ce que** le point de montage (8), sur lequel est monté l'élément ressort (6) dans la région médiane de la poignée (2a) pivotable, est agencé, dans la position terminale inférieure verrouillant la partie de maintien (3), au-dessous d'une ligne (L₁) qui relie le point d'articulation (5) entre la poignée (2a) pivotable et la mâchoire (3a) pivotable avec le point de montage (7) de l'élément ressort (6) dans la région de l'extrémité proximale de la poignée (2b) rigide.

9. Instrument médical de prise et de maintien selon l'une des revendications 1 à 4, **caractérisé en ce que** les deux poignées (2a, 2b) de la manette (2) sont réalisées d'un seul tenant et rigides avec une mâchoire (3a, 3b) respective de la partie de maintien (3), les poignées (2a, 2b) et les mâchoires (3a, 3b) étant montées de manière à pivoter respectivement l'une par rapport à l'autre autour d'un point d'articulation (4) commun en se croisant.

10. Instrument médical de prise et de maintien selon la revendication 9, **caractérisé en ce que** le point d'articulation (8), sur lequel l'élément ressort (6) est monté dans la région médiane d'une poignée (2a), est agencé sur un arc de cercle (K) autour du point d'articulation (4).

11. Instrument médical de prise et de maintien selon la revendication 1, **caractérisé en ce qu'**au moins une mâchoire (3a, 3b) de la partie de maintien (3) est reliée de manière solidaire avec un élément ressort (6) respectif, chaque élément ressort (6) étant agencé sous précontrainte entre le point d'articulation (4), côté distal, pour pivoter au moins une mâchoire (3a, 3b), et un contre-appui (12) rigide, côté proximal, de telle sorte que chaque élément ressort (6) peut être déplacé via une poignée (2a, 2b) respective de la manette (2) entre une position terminale libérant la partie de maintien (3) et une position terminale verrouillant la partie de maintien (3).

12. Instrument médical de prise et de maintien selon la revendication 11, **caractérisé en ce qu'**une mâchoire (3b) de la partie de maintien (3) est réalisée d'un seul tenant et rigide avec une poignée (2b) de la manette (2), et l'autre mâchoire (3a) de la partie de maintien (3) est reliée de manière solidaire avec l'élément ressort (6), le contre-appui (12) de l'élément ressort (6) étant relié de manière rigide avec la poignée (2b) rigide, et l'autre poignée (2a) de la manette (2) étant montée à l'extrémité proximale de la poignée rigide (2b) de manière à pouvoir pivoter autour d'un point d'articulation (11), et la poignée (2a) pivotable étant reliée à l'élément ressort (6).

13. Instrument médical de prise et de maintien selon l'une des revendications 1 à 12, **caractérisé en ce que** l'instrument médical de prise et de maintien est un porte-aiguille (1).

14. Instrument médical de prise et de maintien selon la revendication 1, **caractérisé en ce que** l'instrument médical de prise et de maintien est un instrument à fût tubulaire (14), la manette (2) étant reliée à la partie de maintien (3) via au moins un élément de transmission de force, en particulier une tige tirée/poussée (16).
